# EUROPÄISCHE PATENTSCHRIFT

(11) **EP 3 738 503 B1**
(45) Veröffentlichungstag und Bekanntmachung des Hinweises auf die Patenterteilung: **01.05.2024**
(21) Anmeldenummer: 19174637.9
(22) Anmeldetag: 15.05.2019
(51) Int. Cl.: A61B 5/00, A61B 5/055

(54) **EINSTELLUNG EINER ÜBERTRAGUNGSFREQUENZ EINER PHYSIOLOGISCHEN ÜBERWACHUNGSEINHEIT**
ADJUSTMENT OF A TRANSMISSION FREQUENCY OF A PHYSIOLOGICAL MONITORING UNIT
RÉGLAGE D'UNE FRÉQUENCE DE TRANSMISSION D'UNE UNITÉ DE SURVEILLANCE PHYSIOLOGIQUE

(43) Veröffentlichungstag der Anmeldung: 18.11.2020
(73) Patentinhaber: Siemens Healthineers AG, 91301 Forchheim (DE)
(72) Erfinder: Assmann, Bernd, 90762 Fürth (DE); Rößler, Jürgen, 91086 Aurachtal (DE)
(74) Vertreter: Siemens Healthineers Patent Attorneys

(56) Entgegenhaltungen:
- DE-A1-102008 037 388
- US-A1- 2013 245 387
- US-A1- 2013 289 367
- US-A1- 2015 045 699
- US-A1- 2018 192 955
- US-B1- 8 437 843

## Beschreibung

Die Erfindung betrifft ein Verfahren zu einer Einstellung einer Übertragungsfrequenz einer physiologischen Überwachungseinheit, ein Verfahren zu einem Erfassen eines physiologischen Signals, ein physiologisches Überwachungssystem und eine Programmiereinheit zu einer Einstellung einer Übertragungsfrequenz einer physiologischen Überwachungseinheit.

In einem Magnetresonanzgerät wird üblicherweise der zu untersuchende Körper eines Untersuchungsobjektes, insbesondere eines Patienten, mit Hilfe eines Magneten einem relativ hohen statischen Magnetfeld, beispielsweise von 1,5 oder 3 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse (HF-Pulse), beispielsweise Anregungspulse, ausgesendet, was dazu führt, dass die Kernspins bestimmter, durch diese HF-Pulse resonant angeregter Atome um einen definierten Flipwinkel gegenüber den Magnetfeldlinien des statischen Magnetfelds verkippt werden. Bei der Relaxation der Kernspins werden HF-Signale, so genannte Magnetresonanz-Signale, abgestrahlt, die mittels einer Empfangseinheit umfassend Spuleneinheiten, welche als HF-Antennen ausgebildet sind, empfangen und dann weiterverarbeitet werden. So werden Rohdaten aufgenommen, welche schließlich zu den gewünschten Bilddaten rekonstruiert werden können.

Während der Aufnahme der Rohdaten beeinflusst eine Bewegung des Untersuchungsobjektes die Rohdaten und die daraus zu rekonstruierenden Bilddaten typischerweise negativ. Physiologische Bewegungen, beispielsweise aufgrund von Atmung und/oder eines schlagenden Herzens sind nicht und/oder nur schwer vermeidbar. Beeinflusst eine physiologische Bewegung den Untersuchungsbereich des Untersuchungsobjektes, so ist eine Anpassung und/oder Synchronisation der Aufnahme der Rohdaten mit der physiologischen Bewegung vorteilhaft. Hierzu werden aufgrund der physiologischen Bewegung entstehende physiologische Signale typischerweise mittels einer physiologischen Überwachungseinheit erfasst.

Eine derartige physiologische Überwachungseinheit umfasst typischerweise eine Erfassungseinheit und eine Empfängereinheit. Insbesondere sind die Erfassungseinheit und zumindest ein Teil der Empfängereinheit typischerweise innerhalb eines HF-abgeschirmten Raumes, in dem das Magnetresonanzgerät angeordnet ist, angeordnet. Die Erfassungseinheit ist typischerweise in unmittelbarer Umgebung des Untersuchungsobjektes, vorzugsweise in direktem Kontakt mit diesem, angeordnet. Die Empfängereinheit ist typischerweise zu einer Erfassung eines physiologischen Signals ausgebildet. Die Empfängereinheit ist typischerweise zu einer Auswertung und/oder Verarbeitung des physiologischen Signals ausgebildet und/oder mit einer Steuereinheit zu einer Verarbeitung des physiologischen Signals verbunden, wobei die Steuereinheit auch dazu ausgebildet sein kann, Gradientenpulse und/oder HF-Pulse zu kontrollieren und/oder auszugeben. Daten, insbesondere Daten umfassend von der Erfassungseinheit erfasste physiologische Signale und/oder Daten charakterisierend eine Form und/oder einen Zeitpunkt von der Erfassungseinheit zu erfassender physiologischer Signale, können drahtlos auf einer Übertragungsfrequenz, der Übertragungsfrequenz der physiologischen Überwachungseinheit, zwischen der Erfassungseinheit und der Empfängereinheit übertragen und/oder ausgetauscht werden. Diese Übertragungsfrequenz ist typischerweise fest vorgegeben und/oder voreingestellt. Abhängig von dem Standort des Magnetresonanzgerätes und/oder dem HF-abgeschirmten Raum kann die Übertragungsfrequenz durch Störsignale beeinträchtigt sein und/oder die Übertragungsfrequenz kann innerhalb eines verbotenen Frequenzbandes liegen.

US 2013/245387 A1 offenbart ein ortsabhängiges drahtloses medizinisches Gerät. DE 10 2008 037388 A1 offenbart ein drahtloses Patientenüberwachungssystem zur Verringerung von Interferenzen in einem drahtlosen Sensornetzwerk. US 8 437 843 B1 offenbart ein funkbasiertes EEG-Gerät. US 2018/192955 A1 offenbart einen wiederverwertbaren physiologischen Sensor mit Einwegkomponenten.

Der Erfindung liegt die Aufgabe zugrunde, ein besonders einfaches und genaues Verfahren zu einer Einstellung einer Übertragungsfrequenz einer physiologischen Überwachungseinheit anzugeben. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zu einer Einstellung einer Übertragungsfrequenz einer physiologischen Überwachungseinheit umfassend eine Erfassungseinheit und eine Empfängereinheit, wobei die Erfassungseinheit und die Empfängereinheit drahtlos miteinander verbunden sind und dazu ausgebildet sind, auf der Übertragungsfrequenz Daten auszutauschen, sieht die folgenden Verfahrensschritte vor:
- Bereitstellen der Übertragungsfrequenz durch eine Programmiereinheit,
- Verbinden der Programmiereinheit mit der Erfassungseinheit,
- Übermitteln eines ersten Kodierungssignal repräsentativ für die Übertragungsfrequenz von der Programmiereinheit an die Erfassungseinheit,
- Bereitstellen der Übertragungsfrequenz an die Empfängereinheit.

Die Erfassungseinheit ist dazu ausgebildet, ein physiologisches Signal eines Untersuchungsobjektes zu erfassen.

Die Programmiereinheit umfasst vorzugsweise eine Speichereinheit. Bei Bereitstellen der Übertragungsfrequenz wird die Übertragungsfrequenz vorzugsweise auf der Speichereinheit gespeichert. Das Bereitstellen der Übertragungsfrequenz kann durch Vorgabe eines Benutzers erfolgen. Die Programmiereinheit weist vorzugsweise eine erste Schnittstelle auf, über welche erste Schnittstelle die Vorgabe der Übertragungsfrequenz, beispielsweise durch einen Benutzer, erfolgen kann. Die Programmiereinheit ist typischerweise dazu ausgebildet, eine Information umfassend die Übertragungsfrequenz zu empfangen und/oder zu verarbeiten und/oder das erste Kodierungssignal und/oder ein zweites Kodierungssignal auszugeben. Die Programmiereinheit umfasst vorzugsweise eine zweite Schnittstelle zum Verbinden der Programmiereinheit mit der Erfassungseinheit und/oder der Empfängereinheit. Die erste Schnittstelle und die zweite Schnittstelle können voneinander verschieden sein. Die erste Schnittstelle und die zweite Schnittstelle können gemeinsam in die Programmiereinheit integriert sein.

Die Empfängereinheit ist typischerweise mit einem medizinischen Bildgebungsgerät und/oder einer Steuerungseinheit für ein medizinisches Bildgebungsgerät wie beispielsweise ein Magnetresonanzgerät verbunden. Die Empfängereinheit ist typischerweise dazu ausgebildet, einer Steuerungseinheit für ein medizinisches Bildgebungsgerät das erste Kodierungssignal und/oder die Übertragungsfrequenz zu übermitteln. Das Kodierungssignal repräsentativ für die Übertragungsfrequenz umfasst typischerweise die Übertragungsfrequenz. Die Übertragungsfrequenz ist typischerweise eine vorab definierte Frequenz. Die Übertragungsfrequenz ist typischerweise derart definiert, dass diese außerhalb eines verbotenen Frequenzbandes liegt und/oder sich von einer Frequenz eines lokalen Störsignals unterscheidet. Ein lokales Störsignal kann von einem individuellen Standort des Magnetresonanzgerätes abhängig sein. Das Bereitstellen der Übertragungsfrequenz kann eine Vorgabe durch einen Benutzer und/oder ein automatisches Ermitteln umfassen. Das Übermitteln eines ersten Kodierungssignal repräsentativ für die Übertragungsfrequenz umfasst typischerweise ein Speichern der Übertragungsfrequenz auf der Erfassungseinheit. Bei Benutzung der physiologischen Überwachungseinheit werden Daten zwischen der Erfassungseinheit und der Empfängereinheit typischerweise auf Übertragungsfrequenz ausgetauscht. Die an die Empfängereinheit bereitgestellte Übertragungsfrequenz entspricht der vom Kodierungssignal umfassten Übertragungsfrequenz.

Das erfindungsgemäße Verfahren ermöglicht eine kostengünstige Änderung der Übertragungsfrequenz einer physiologischen Überwachungseinheit. Insbesondere kann die Übertragungsfrequenz einer konventionellen physiologischen Überwachungseinheit softwarebasiert und/oder ohne Änderung der Hardware geändert werden. Zusätzlich kann auf eine Änderung der Steuerungseinheit des Magnetresonanzgerätes und/oder eine Änderung einer darauf auszuführenden Steuerungssoftware verzichtet werden, da die Änderung der Übertragungsfrequenz direkt an der physiologischen Überwachungseinheit vorgenommen werden kann. Zusätzlich kann auf eine separate Benutzeroberfläche und/oder Anzeige an einer zur Bedienung des Magnetresonanzgerätes ausgebildeten Steuerungseinheit und/oder dazugehörigen Anzeigeeinheit verzichtet werden. Dadurch kann eine fehlerhafte Bedienung durch nicht autorisiertes und/oder qualifiziertes Personal vermieden werden.

Das erfindungsgemäße Verfahren stellt sicher, dass die Erfassungseinheit und die Empfängereinheit auf der Übertragungsfrequenz, also auf derselben Frequenz, Daten drahtlos übertragen, also senden und empfangen. Dadurch ist eine drahtlose Übertragung von Daten jederzeit gewährleistet, insbesondere im Betrieb der physiologischen Überwachungseinheit.

Eine Ausführungsform des Verfahrens sieht vor, dass das erste Kodierungssignal ein Triggersignal umfasst, und das Übermitteln des ersten Kodierungssignals eine Signalisierung einer beabsichtigten Änderung der Übertragungsfrequenz an die Erfassungseinheit durch das Triggersignal umfasst.

In dieser Ausführungsform der Erfindung ist die Erfassungseinheit dazu ausgebildet, das Triggersignal bei Übertragung des ersten Kodierungssignals zu erkennen. Die Erfassungseinheit ist dazu ausgebildet, bei Erkennen des Triggersignals einen Status anzunehmen, in welchem eine Änderung der Übertragungsfrequenz zulässig ist und eine Aufnahme physiologischer Signale unterbrochen wird. Die Übertragung des Triggersignals initiiert typischerweise diesen Status. Es ist möglich, dass nach Erkennen des Triggersignals zur Initiierung dieses Status eine Bestätigung durch einen Benutzer erforderlich ist, bevor die Übertragungsfrequenz an der Erfassungseinheit tatsächlich geändert wird.

Das Triggersignal ist vorzugsweise eine definierte Signalfolge. Das erste Kodierungssignal ist umfasst vorzugsweise zusätzlich zum Triggersignal ein weiteres Signal repräsentativ für die Übertragungsfrequenz. Das Triggersignal und das weitere Signal können zumindest teilweise kombiniert sein. Das Triggersignal und/oder das erste Kodierungssignal ist derart gewählt, dass es sich von einem physiologischen Signal, welches durch die Erfassungseinheit erfassbar ist, unterscheidet. Ein physiologisches Signal kann beispielsweise ein Signal repräsentativ für ein Elektrokardiogramm und/oder einen Puls eines Menschen sein. Diese Ausführungsform ermöglicht eine eindeutige Kennzeichnung des Beginns der Übermittlung des ersten Kodierungssignal repräsentativ für die Übertragungsfrequenz von der Programmiereinheit an die Erfassungseinheit. Dadurch kann das Verfahren besonders robust ausgeführt werden.

Eine Ausführungsform des Verfahrens sieht vor, dass die Erfassungseinheit eine Signaleinheit umfasst, welche Signaleinheit das Verbinden der Programmiereinheit mit der Erfassungseinheit indiziert. Die Signaleinheit kann beispielsweise als Leuchte, insbesondere als LED ausgebildet sein. Die Signaleinheit kann alternativ oder zusätzlich audiologisch ausgebildet sein.

Die Signaleinheit signalisiert vorzugsweise das Übermitteln des ersten Kodierungssignal an die Erfassungseinheit bei Ausführung des Verfahrens gemäß dieser Ausführungsform. Die Signaleinheit signalisiert vorzugsweise den Status, in welchem eine Änderung der Übertragungsfrequenz zulässig ist und vorzugsweise eine Aufnahme physiologischer Signale nicht zulässig ist und/oder unterbrochen wird. Hierzu indiziert die Signaleinheit vorzugsweise ein erstes Signal. Die Signaleinheit ist vorzugsweise ebenso dazu ausgebildet, den Betrieb der physiologischen Überwachungseinheit bei Erfassung physiologischer Signale zu indizieren. Hierzu indiziert die Signaleinheit vorzugsweise ein zweites Signal. Das erste Signal unterscheidet sich vorzugsweise vom zweiten Signal. Diese Ausführungsform ermöglicht eine Rückmeldung an einen das Verfahren ausführenden Benutzer, dass die Übertragungsfrequenz geändert wird und/oder geändert wurde.

Eine erste Alternative des erfindungsgemäßen Verfahrens sieht vor, dass das Bereitstellen der Übertragungsfrequenz an die Empfängereinheit mittels Eingabe durch einen Benutzer erfolgt.

Gemäß dieser Ausführungsform weist das Magnetresonanzgerät und/oder eine Steuerungseinheit für das Magnetresonanzgerät und/oder für die physiologischen Überwachungseinheit vorzugsweise ein User Interface auf, mittels dem geschultes Personal und/oder ein Benutzer die Übertragungsfrequenz an die Empfängereinheit übermitteln kann. Dies ist eine kostengünstige Lösung zur Änderung der Übertragungsfrequenz, welche gemäß dem beanspruchten Verfahren der Empfängereinheit und der Erfassungseinheit übermittelt wird.

Eine zweite Alternative des erfindungsgemäßen Verfahrens sieht vor, dass die Empfängereinheit einen Eingang und eine Empfängersteuereinheit umfasst und das Bereitstellen der Übertragungsfrequenz an die Empfängereinheit
- ein Verbinden der Programmiereinheit mit dem Eingang, und
- ein Übermitteln eines zweiten Kodierungssignal repräsentativ für die Übertragungsfrequenz von der Programmiereinheit an die Empfängersteuereinheit umfasst.

Der Eingang umfasst vorzugsweise eine Anschlussstelle, an die die Programmiereinheit passgenau anschließbar ist. Der Eingang kann auch eine Schnittstelle, beispielsweise eine drahtlose Schnittstelle, beispielsweise für eine Bluetooth-Verbindung, sein. Die Empfängersteuereinheit ist vorzugsweise dazu ausgebildet, von der Erfassungseinheit an die Empfängereinheit übertragene Daten zu verarbeiten und/oder Daten an die Erfassungseinheit zu übertragen und/oder dies vorzugsbereiten. Dieser Ausführungsform ermöglicht eine softwarebasierte Einstellung der Übertragungsfrequenz an der Empfängereinheit und erfordert typischerweise keine Änderung der Hardware der Empfängereinheit. Dadurch ist eine kostengünstige Ausführung des Verfahrens möglich, da insbesondere keine Nachrüstung vorhandener physiologischer Überwachungseinheiten erforderlich ist. Diese Ausführungsform kann mittels der Programmiereinheit einfach, auch von einem ungeschulten Benutzer, ausgeführt werden.

Das zweite Kodierungssignal umfasst vorzugsweise ein Triggersignal und das Übermitteln des zweiten Kodierungssignals umfasst eine Signalisierung einer beabsichtigten Änderung der Übertragungsfrequenz an die Empfängereinheit durch das Triggersignal. Die Empfängereinheit ist typischerweise dazu ausgebildet, das Triggersignal bei Übertragung des zweiten Kodierungssignals zu erkennen. Die Empfängereinheit ist typischerweise dazu ausgebildet, bei Erkennen des Triggersignals einen Status anzunehmen, in welchem eine Änderung der Übertragungsfrequenz zulässig ist und vorzugsweise eine Aufnahme physiologischer Signale nicht zulässig ist und/oder unterbrochen wird. Die Übertragung des Triggersignals initiiert typischerweise diesen Status. Es ist möglich, dass nach Erkennen des Triggersignals zur Initiierung dieses Status eine Bestätigung durch einen Benutzer erforderlich ist, bevor die Übertragungsfrequenz an der Empfängereinheit tatsächlich geändert wird. Das Triggersignal ist vorzugsweise eine definierte Signalfolge. Das zweite Kodierungssignal ist umfasst vorzugsweise zusätzlich zum Triggersignal ein weiteres Signal repräsentativ für die Übertragungsfrequenz. Das Triggersignal und das weitere Signal können zumindest teilweise kombiniert sein. Das Triggersignal und/oder das zweite Kodierungssignal ist derart gewählt, dass es sich von einem physiologischen Signal, welches durch die Erfassungseinheit erfassbar ist, unterscheidet. Diese Ausführungsform ermöglicht eine eindeutige Kennzeichnung des Beginns der Übermittlung des zweiten Kodierungssignal repräsentativ für die Übertragungsfrequenz von der Programmiereinheit an die Empfängereinheit. Dadurch kann das Verfahren besonders robust ausgeführt werden.

Das erfindungsgemäße Verfahren sieht vor, dass die Erfassungseinheit eine Sensoreinheit und eine Erfassungssteuereinheit umfasst, die Sensoreinheit zur Aufnahme physiologischer Signale eines Untersuchungsobjekts ausgebildet ist, die Programmiereinheit eine Kopplungseinheit umfasst und das Verbinden der Programmiereinheit mit der Erfassungseinheit ein Anschließen der Kopplungseinheit an die Sensoreinheit umfasst. Die Sensoreinheit und die Kopplungseinheit sind derart ausgebildet, dass das Anschließen der Kopplungseinheit an die Sensoreinheit passgenau erfolgt. Dies ermöglicht eine intuitive und/oder einfache Ausführung des Verfahrens gemäß dieser Ausführungsform.

Eine Ausführungsform des Verfahrens sieht vor, dass die Erfassungssteuereinheit dazu ausgebildet ist, das erste Kodierungssignal von physiologischen Signalen zu unterscheiden. Das erste Kodierungssignal repräsentativ für die Übertragungsfrequenz ist vorzugsweise derart gewählt, dass es nicht durch eine physiologische Eigenschaft eines Menschen erzeugbar ist. Dies kann beispielsweise ein besonders intensives Signal und/oder ein zeitlicher Verlauf sein, der keinen natürlichen Ursprung haben kann. Das Kodierungssignal ist vorzugsweise derart gewählt, dass es sich von jedem möglichen menschlichem Blutfluss und/oder menschlichen Elektrokardiogramm unterscheidet. Diese Ausführungsform ermöglicht eine eindeutige Kennzeichnung der Übermittlung des ersten Kodierungssignal repräsentativ für die Übertragungsfrequenz von der Programmiereinheit an die Erfassungseinheit. Dadurch kann das Verfahren besonders robust ausgeführt werden.

Eine Ausführungsform des Verfahrens sieht vor, dass die Sensoreinheit zumindest zwei Elektroden umfasst und dazu ausgebildet ist, ein Elektrokardiogramm zu erfassen, die Kopplungseinheit zumindest einen Pin umfasst, und das Anschließen der Kopplungseinheit an die Sensoreinheit ein Verbinden des zumindest einen Pins mit einer der zumindest zwei Elektroden umfasst. Die Sensoreinheit ist dazu ausgebildet, mittels der zumindest zwei Elektroden ein Elektrokardiogramm zu erfassen, insbesondere bei Positionierung der Elektroden an einem Untersuchungsobjekt, insbesondere einem Patienten. Der zumindest eine Pin und/oder die zumindest zwei Elektroden sind typischerweise elektrisch leitfähig. Vorzugsweise kann genau ein Pin mit jeweils einer der zumindest zwei Elektroden elektrisch leitend und/oder passgenau verbunden werden. Das ersten Kodierungssignal wird typischerweise zwischen dem zumindest einem Pin und einer der zumindest zwei Elektroden übermittelt. Die Kopplungseinheit kann auch zwei oder mehrere Pins umfassen.

Der Vorteil dieser Ausführungsform liegt darin, dass aufgrund der Ausgestaltung der Programmiereinheit diese an eine existierende Schnittstelle der Erfassungseinheit, gebildet durch die Sensoreinheit in Form von Elektroden zur Erfassung eines Elektrokardiogramms, anschließbar ist. Dadurch kann die Programmiereinheit einfach verwendet werden und das Verfahren besonders einfach ausgeführt werden.

Eine Ausführungsform des Verfahrens sieht vor, dass das Übermitteln des ersten Kodierungssignals das Ausgeben eines zeitlich veränderten elektrischen Stromes umfasst. Das erste Kodierungssignal hat demnach vorzugsweise die gleiche physikalische Grundlage wie das physiologische Signal. Das erste Kodierungssignal unterscheidet sich vorzugsweise in der Ausgestaltung von einem physiologischen Signal, beispielsweise in der zeitlichen Abfolge und/oder Intensität des elektrischen Stromes, damit eine Unterscheidung zwischen dem ersten Kodierungssignal und einem physiologischen Signal möglich ist. Dies ermöglicht eine besonders effiziente Verwendung vorhandener Elektroden.

Eine Ausführungsform des Verfahrens sieht vor, dass die Sensoreinheit dazu ausgebildet ist, einen Puls eines Untersuchungsobjektes zu erfassen. Der Puls ist ein regelmäßig mittels einer physiologischen Überwachungseinheit überwachtes physiologisches Signal. Das Verfahren gemäß dieser Ausführungsform kann demnach mit einer häufig verwendeten physiologischen Überwachungseinheit ausgeführt werden. Dies ermöglicht eine besonders effiziente Verwendung einer vorhandenen Sensoreinheit.

Eine Ausführungsform des Verfahrens sieht vor, dass die Sensoreinheit einen Fingerclip umfasst, die Kopplungseinheit eine Form eines Fingers aufweist und das Verbinden der Programmiereinheit mit der Erfassungseinheit ein Umschließen der Kopplungseinheit durch den Fingerclip umfasst.

Gemäß dieser Ausführungsform weist die Kopplungseinheit eine derartige Form auf, dass diese durch den Fingerclip zumindest teilweise passgenau umschließbar ist. Das ersten Kodierungssignal wird typischerweise zwischen dem Fingerclip und einer der Kopplungseinheit übermittelt. Der Vorteil dieser Ausführungsform liegt darin, dass aufgrund der Ausgestaltung der Programmiereinheit diese an eine existierende Schnittstelle der Erfassungseinheit, gebildet durch die Sensoreinheit in Form eines Fingerclips zur Erfassung eines Pulsschlages des Untersuchungsobjektes, anschließbar ist. Dadurch kann die Programmiereinheit einfach verwendet werden und das Verfahren besonders einfach ausgeführt werden. Die Sensoreinheit, insbesondere der Fingerclip, umfasst vorzugsweise ein Glasfaserkabel. Die Kopplungseinheit umfasst vorzugsweise ein Glasfaserkabel. Bei Anschluss der Kopplungseinheit and die Sensoreinheit erfolgt vorzugsweise ein Verbinden der jeweiligen Glasfaserkabel, insbesondere deren Enden, miteinander. Dies ermöglicht eine besonders effiziente Verwendung einer vorhandenen Sensoreinheit und resultiert in einem robusten Verfahren.

Eine Ausführungsform des Verfahrens sieht vor, dass das Übermitteln des ersten Kodierungssignals das Ausgeben eines zeitlich veränderten Lichtsignals umfasst. Die Kopplungseinheit in Form eines Fingers kann eine variable Lichtdurchlässigkeit aufweisen, welche beispielsweise von der Programmiereinheit gemäß des ersten Kodierungssignals verändert werden kann. Das erste Kodierungssignals kann demnach ein zeitlich verändertes Lichtsignal umfassen. Auch ein vom ersten Kodierungssignal umfasstes Triggersignal kann ein zeitlich verändertes Lichtsignal sein. Diese Ausführungsform ermöglicht eine kostengünstige Benutzung der vorhandenen Sensoreinheit.

Das erfindungsgemäße Verfahren zu einem Erfassen eines physiologischen Signals mittels einer physiologischen Überwachungseinheit umfassend eine Erfassungseinheit und eine Empfängereinheit, wobei die Erfassungseinheit und die Empfängereinheit drahtlos miteinander verbunden sind und dazu ausgebildet sind, auf der Übertragungsfrequenz Daten auszutauschen, sieht die folgenden Verfahrensschritten vor:
- Detektion des physiologischen Signals mittels der Erfassungseinheit,
- Drahtloses Übertragen des physiologischen Signals von der Erfassungseinheit auf die Empfängereinheit auf der Übertragungsfrequenz,
- Verarbeitung des physiologischen Signals durch die Empfängereinheit,
wobei die Übertragungsfrequenz der physiologischen Überwachungseinheit gemäß einer oben aufgeführten Ausführungsformen eingestellt wird. Des Weiteren geht die Erfindung aus von einem physiologischen Überwachungssystem umfassend eine Programmiereinheit und eine physiologische Überwachungseinheit umfassend eine Erfassungseinheit und eine Empfängereinheit, welche zu einer Ausführung eines erfindungsgemäßen Verfahrens zu einer Einstellung einer Übertragungsfrequenz ausgelegt ist.

Des Weiteren geht die Offenbarung aus von einer Programmiereinheit umfassend eine Kopplungseinheit und eine Programmiersteuereinheit, welche dazu ausgebildet ist, eine Übertragungsfrequenz einer physiologischen Überwachungseinheit umfassend eine Erfassungseinheit und eine Empfängereinheit, welche drahtlos miteinander verbunden sind und dazu ausgebildet sind, auf der Übertragungsfrequenz Daten auszutauschen, einzustellen, wobei die Programmiersteuereinheit zu einer Bereitstellung der Übertragungsfrequenz und zu einer Erzeugung eines ersten Kodierungssignals repräsentativ für die Übertragungsfrequenz ausgebildet ist, und die Kopplungseinheit zu einem Verbinden mit der Erfassungseinheit und zu einem Übermitteln des ersten Kodierungssignal an die Erfassungseinheit ausgebildet ist. Die Vorteile des Verfahrens zu einem Erfassen eines physiologischen Signals, des physiologischen Überwachungssystems und der Programmiereinheit entsprechen im Wesentlichen den Vorteilen des erfindungsgemäßen Verfahrens zu einer Einstellung einer Übertragungsfrequenz einer physiologischen Überwachungseinheit, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: ein erfindungsgemäßes physiologisches Überwachungssystem in einer schematischen Darstellung in einer ersten Ausführungsform,
- Fig. 2: ein erfindungsgemäßes physiologisches Überwachungssystem in einer schematischen Darstellung in einer zweiten Ausführungsform,
- Fig. 3: ein erfindungsgemäßes physiologisches Überwachungssystem in einer schematischen Darstellung in einer dritten Ausführungsform,
- Fig. 4: eine erfindungsgemäße Programmiereinheit in einer schematischen Darstellung,
- Fig. 5: ein Ablaufdiagramm einer ersten Ausführungsform eines erfindungsgemäßen Verfahrens zu einer Einstellung einer Übertragungsfrequenz,
- Fig. 6: ein Ablaufdiagramm einer zweiten Ausführungsform eines erfindungsgemäßen Verfahrens zu einer Einstellung einer Übertragungsfrequenz, und
- Fig. 7: ein Ablaufdiagramm eines erfindungsgemäßen Verfahrens zu einem Erfassen eines physiologischen Signals.

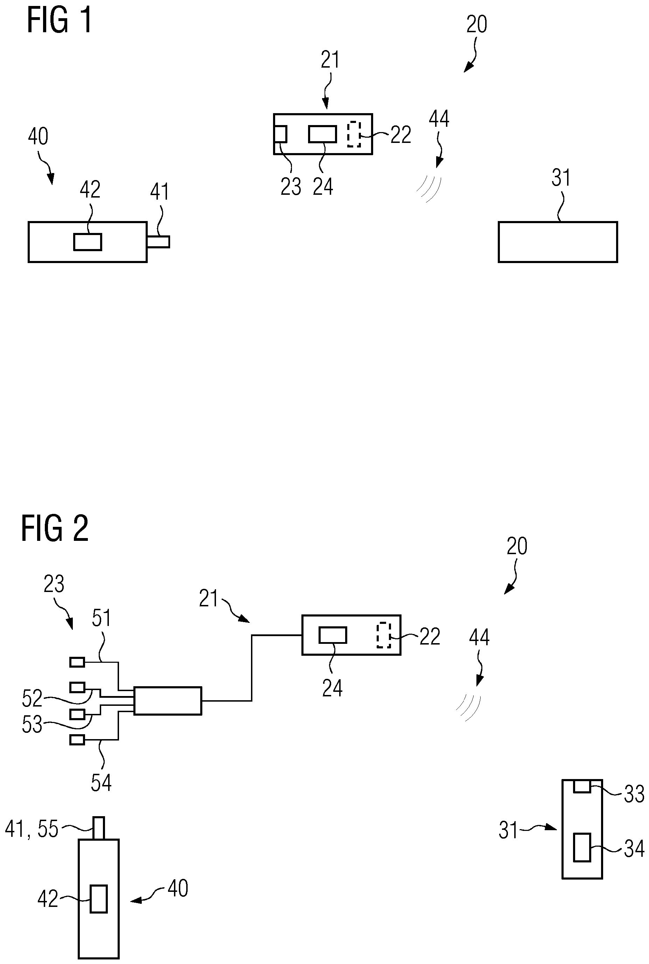

Figur 1 zeigt ein erfindungsgemäßes physiologisches Überwachungssystem in einer schematischen Darstellung in einer ersten Ausführungsform.

Das physiologische Überwachungssystem umfasst eine Programmiereinheit 40 und eine physiologische Überwachungseinheit 20 umfassend eine Erfassungseinheit 21 und eine Empfängereinheit 31, welche zu einer Ausführung des erfindungsgemäßen Verfahrens zu einer Einstellung einer Übertragungsfrequenz 44 der physiologischen Überwachungseinheit 20 ausgelegt ist. Die Erfassungseinheit 21 und die Empfängereinheit 31 sind drahtlos miteinander verbunden und dazu ausgebildet, auf der Übertragungsfrequenz 44 Daten auszutauschen.

Die Erfassungseinheit 21 umfasst vorzugsweise eine Signaleinheit 22, welche ein Verbinden der Programmiereinheit 40 mit der Erfassungseinheit 21 und/oder ein Erkennen einer beabsichtigten Änderung der Übertragungsfrequenz mittels der Programmiereinheit 40 indiziert. Die Signaleinheit 22 kann beispielsweise derart ausgelegt sein, dass die Indizierung visuell oder akustisch erfolgt. Die Signaleinheit kann beispielsweise in Form eines Lautsprechers und/oder einer LED ausgebildet sein. Die Signaleinheit 22 signalisiert vorzugsweise weitere Funktionen der Erfassungseinheit 21, beispielsweise beim Erfassen eines physiologischen Signals des Untersuchungsobjektes.

Figur 2 zeigt ein erfindungsgemäßes physiologisches Überwachungssystem in einer schematischen Darstellung in einer zweiten Ausführungsform. Die zweite Ausführungsform ist eine optionale Erweiterung der ersten Ausführungsform. Die Empfängereinheit 31 umfasst einen Eingang 33 und eine Empfängersteuereinheit 34. Der Eingang 33 ist derart ausgestaltet, dass die Programmiereinheit 40, insbesondere eine von der Programmiereinheit 40 umfasste Kopplungseinheit 41, mit dem Eingang 33 verbindbar ist. Vorzugsweise sind die Kopplungseinheit 41 und der Eingang 33 derart aufeinander abgestimmt, dass die Kopplungseinheit 41 und der Eingang 33 eine passgenaue und/oder lösbare Verbindung und/oder Steckverbindung bilden können.

Die Erfassungseinheit 21 umfasst eine Sensoreinheit 23, welche zur Aufnahme physiologischer Signale eines Untersuchungsobjekts ausgebildet ist, und eine Erfassungssteuereinheit 24. Die von der Programmiereinheit 40 umfasste Kopplungseinheit 41 ist zu einem Verbinden der Programmiereinheit 40 mit der Erfassungseinheit 21 durch ein Anschließen der Kopplungseinheit 41 an die Sensoreinheit 23 ausgebildet. Die Sensoreinheit 23 umfasst zumindest zwei, in der dargestellten Ausführungsform vier Elektroden 51, 52, 53, 54, wodurch die Sensoreinheit 23 dazu ausgebildet ist, ein Elektrokardiogramm zu erfassen. Die Kopplungseinheit 41 umfasst zumindest einen Pin 55. Der zumindest eine Pin 55 ist dazu ausgebildet, an eine der vier Elektroden 51, 52, 53, 54 angeschlossen zu werden. Die Kopplungseinheit 41 kann mehrere Pins umfassen, wobei die Pins derart ausgestaltet sind, dass jeweils ein Pin mit einer von der Sensoreinheit 23 umfassten Elektrode verbindbar ist.

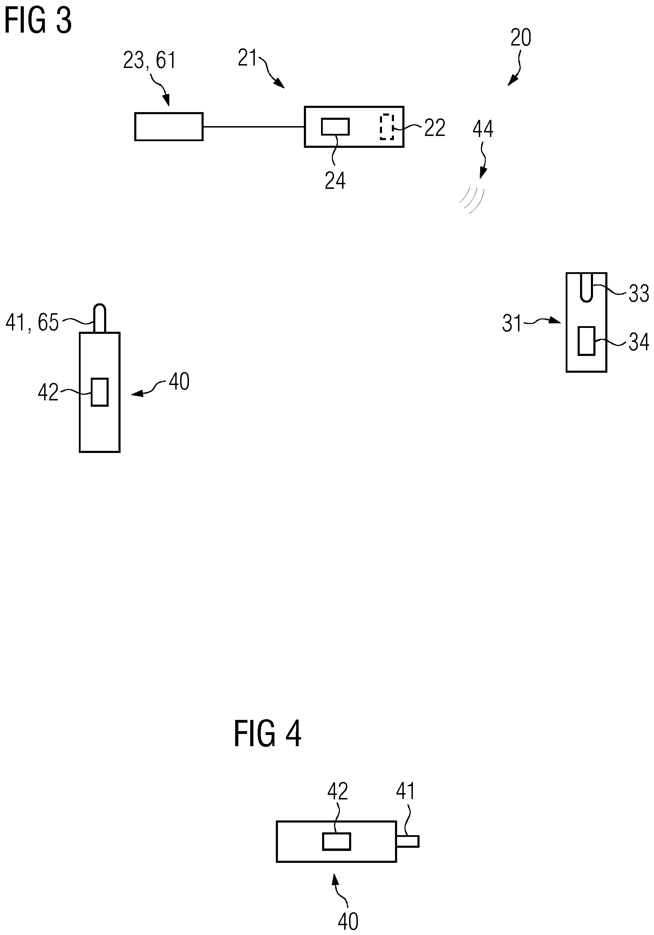

Figur 3 zeigt ein erfindungsgemäßes physiologisches Überwachungssystem in einer schematischen Darstellung in einer dritten Ausführungsform. Die dritte Ausführungsform ist eine optionale Erweiterung der ersten Ausführungsform und typischerweise alternativ zur zweiten Ausführungsform.

Die Erfassungseinheit 21 umfasst eine Sensoreinheit 23 und eine Erfassungssteuereinheit 24, wobei die Sensoreinheit 23 zum Erfassen eines Pulses eines Untersuchungsobjektes ausgebildet ist. Dabei umfasst die Sensoreinheit 23 einen Fingerclip 61, welcher dazu ausgebildet ist, den Puls des Untersuchungsobjektes anhand dessen Finger zu ermitteln. Die Programmiereinheit 40 umfasst eine Kopplungseinheit 41, 65 in Form eines Fingers und das Verbinden der Programmiereinheit 40 mit der Erfassungseinheit 21 umfasst ein Anschließen der Kopplungseinheit 41 an die Sensoreinheit 23 durch den Fingerclip 61.

Die Empfängereinheit 31 umfasst einen Eingang 33 und eine Empfängersteuereinheit 34. Der Eingang 33 ist derart ausgestaltet, dass die Programmiereinheit 40, insbesondere eine von der Programmiereinheit 40 umfasste Kopplungseinheit 41, 65 in Form eines Fingers, mit dem Eingang 33 verbindbar ist. Vorzugsweise sind die Kopplungseinheit 41 und der Eingang 33 derart aufeinander abgestimmt, dass die Kopplungseinheit 41 und der Eingang 33 eine passgenaue und/oder lösbare Verbindung und/oder Steckverbindung bilden können, der Eingang 33 also zur Aufnahme der Kopplungseinheit 41, 65 in Form eines Fingers ausgelegt ist.

Figur 4 zeigt eine erfindungsgemäße Programmiereinheit 40 in einer schematischen Darstellung. Die Programmiereinheit 40 umfasst eine Kopplungseinheit 41 und eine Programmiersteuereinheit 42, welche dazu ausgebildet ist, eine Übertragungsfrequenz 44 einer physiologischen Überwachungseinheit 20 umfassend eine Erfassungseinheit 21 und eine Empfängereinheit 31, welche drahtlos miteinander verbunden sind und dazu ausgebildet sind, auf der Übertragungsfrequenz 44 Daten auszutauschen, einzustellen. Die Programmiersteuereinheit 42 ist zu einer Bereitstellung der Übertragungsfrequenz 44 und zu einer Erzeugung eines ersten Kodierungssignals repräsentativ für die Übertragungsfrequenz 44 ausgebildet und die Kopplungseinheit 41 ist zu einem Verbinden mit der Erfassungseinheit 21 und zu einem Übermitteln des ersten Kodierungssignal an die Erfassungseinheit 21 ausgebildet ist. Die Kopplungseinheit 41 kann beispielsweise gemäß Figur 3 in Form eines Fingers 65 oder gemäß Figur 2 in Form eines Pins 55 ausgebildet sein. Zudem kann die Kopplungseinheit 41 dazu ausgebildet sein, mit einem Eingang 33 der Empfängereinheit 31 verbunden zu werden.

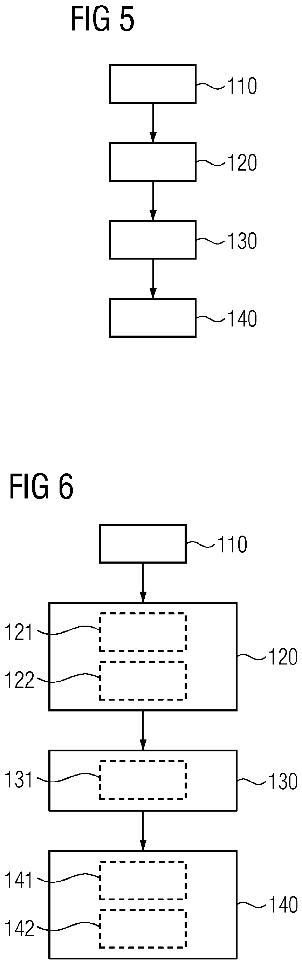

Figur 5 zeigt ein Ablaufdiagramm einer ersten Ausführungsform eines erfindungsgemäßen Verfahrens zu einer Einstellung der Übertragungsfrequenz 44. Das Verfahren beginnt mit Verfahrensschritt 110, dem Bereitstellen der Übertragungsfrequenz 44 durch die Programmiereinheit 40. In Verfahrensschritt 120 erfolgt das Verbinden der Programmiereinheit 40 mit der Erfassungseinheit 21, wonach in Verfahrensschritt 130 das Übermitteln eines ersten Kodierungssignal repräsentativ für die Übertragungsfrequenz 44 von der Programmiereinheit 40 an die Erfassungseinheit 21 erfolgt. In Verfahrensschritt 140 erfolgt das Bereitstellen der Übertragungsfrequenz 44 an die Empfängereinheit 31. Verfahrensschritt 140 kann beispielsweise mittels Eingabe durch einen Benutzer erfolgen.

Figur 6 zeigt ein Ablaufdiagramm einer zweiten Ausführungsform eines erfindungsgemäßen Verfahrens zu einer Einstellung einer Übertragungsfrequenz 44. Die zweite Ausführungsform ist eine optionale Erweiterung der ersten Ausführungsform.

Umfasst die Erfassungseinheit 21 eine Sensoreinheit 23 und eine Erfassungssteuereinheit 24, und die Programmiereinheit 40 eine Kopplungseinheit 41, so kann Verfahrensschritt 120 Verfahrensschritt 121, ein Anschließen der Kopplungseinheit an die Sensoreinheit, umfassen.

Sofern die Sensoreinheit 23 zumindest zwei Elektroden 51, 52, 53, 54 umfasst und dazu ausgebildet ist, ein Elektrokardiogramm zu erfassen, und die Kopplungseinheit 41 zumindest einen Pin 55 umfasst, kann Verfahrensschritt 121, das Anschließen der Kopplungseinheit 41 an die Sensoreinheit 23, ein Verbinden des zumindest einen Pins 55 mit einer der zumindest zwei Elektroden 51, 52, 53, 54 umfassen. Sofern die Sensoreinheit 23 einen Fingerclip 61 umfasst und die Kopplungseinheit 41 eine Form eines Fingers 65 aufweist, kann Verfahrensschritt 120, insbesondere Verfahrensschritt 121, ein Umschließen der Kopplungseinheit 41 durch den Fingerclip umfassen.

Die Erfassungseinheit 21 kann eine Signaleinheit 22 umfassen, welche gemäß Verfahrensschritt 122 bei Verfahrensschritt 120 und/oder bei Verfahrensschritt 130 ein Signal ausgibt und/oder ein Erkennen der beabsichtigten Änderung der Übertragungsfrequenz indiziert.

Sofern das erste Kodierungssignal ein Triggersignal umfasst, kann Verfahrensschritt 130, das Übermitteln des ersten Kodierungssignals, Verfahrensschritt 131, eine Signalisierung einer beabsichtigten Änderung der Übertragungsfrequenz 44 an die Erfassungseinheit 21 durch das Triggersignal umfassen. Die Erfassungssteuereinheit 24 ist vorzugsweise dazu ausgebildet, das erste Kodierungssignal von physiologischen Signalen zu unterscheiden. Verfahrensschritt 130 kann beispielsweise ein Ausgeben eines zeitlich veränderten elektrischen Stromes und/oder Lichtsignals umfassen.

Sofern die Empfängereinheit 31 einen Eingang 33 und eine Empfängersteuereinheit 34 umfasst, kann Verfahrensschritt 140, das Bereitstellen der Übertragungsfrequenz 44 an die Empfängereinheit 31, die folgenden Verfahrensschritte umfassen:
Verfahrensschritt 141, das Verbinden der Programmiereinheit 40 mit dem Eingang 31, und Verfahrensschritt 142, das Übermitteln eines zweiten Kodierungssignal repräsentativ für die Übertragungsfrequenz 44 von der Programmiereinheit 40 an die Empfängersteuereinheit 34.

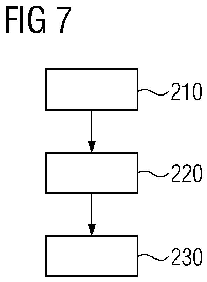

Figur 7 zeigt ein Ablaufdiagramm eines erfindungsgemäßen Verfahrens zu einem Erfassen eines physiologischen Signals mittels der physiologischen Überwachungseinheit 20 umfassend eine Erfassungseinheit 21 und eine Empfängereinheit 31, wobei die Erfassungseinheit 21 und die Empfängereinheit 31 drahtlos miteinander verbunden sind und dazu ausgebildet sind, auf der Übertragungsfrequenz 44 Daten auszutauschen. Die Übertragungsfrequenz 44 wurde gemäß dem erfindungsgemäßen Verfahren eingestellt. Gemäß Verfahrensschritt 210 erfolgt die Detektion des physiologischen Signals mittels der Erfassungseinheit 21. Gemäß Verfahrensschritt 220 erfolgt das drahtlose Übertragen des physiologischen Signals von der Erfassungseinheit 21 auf die Empfängereinheit 31 auf der Übertragungsfrequenz 44. Gemäß Verfahrensschritt 230 erfolgt die Verarbeitung des physiologischen Signals durch die Empfängereinheit 31.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer Einstellung einer Übertragungsfrequenz einer physiologischen Überwachungseinheit umfassend eine Erfassungseinheit und eine Empfängereinheit,
wobei die Erfassungseinheit und die Empfängereinheit drahtlos miteinander verbunden sind und dazu ausgebildet sind, auf der Übertragungsfrequenz Daten auszutauschen,
die Erfassungseinheit eine Sensoreinheit und eine Erfassungssteuereinheit umfasst, und
die Sensoreinheit zur Aufnahme physiologischer Signale eines Untersuchungsobjekts ausgebildet ist,
mittels einer Programmiereinheit umfassend eine Kopplungseinheit gemäß den folgenden Verfahrensschritten:
- Bereitstellen der Übertragungsfrequenz durch die Programmiereinheit,
- Verbinden der Programmiereinheit mit der Erfassungseinheit umfassend ein Anschließen der Kopplungseinheit an die Sensoreinheit,
- Übermitteln eines ersten Kodierungssignal repräsentativ für die Übertragungsfrequenz von der Programmiereinheit an die Erfassungseinheit,
- Bereitstellen der Übertragungsfrequenz an die Empfängereinheit mittels Eingabe durch einen Benutzer oder gemäß den folgenden Verfahrensschritten:
- Verbinden der Programmiereinheit mit einem von der Empfängereinheit umfassten Eingang, und
- Übermitteln eines zweiten Kodierungssignals repräsentativ für die Übertragungsfrequenz von der Programmiereinheit an eine von der Empfängereinheit umfasste Empfängersteuereinheit,
wobei die Sensoreinheit zumindest zwei Elektroden umfasst und dazu ausgebildet ist, ein Elektrokardiogramm zu erfassen, die Kopplungseinheit zumindest einen Pin umfasst,
und das Anschließen der Kopplungseinheit an die Sensoreinheit ein Verbinden des zumindest einen Pins mit einer der zumindest zwei Elektroden umfasst.

2. Verfahren nach Anspruch 1,
wobei das erste Kodierungssignal ein Triggersignal umfasst, und das Übermitteln des ersten Kodierungssignals eine Signalisierung einer beabsichtigten Änderung der Übertragungsfrequenz an die Erfassungseinheit durch das Triggersignal umfasst.

3. Verfahren nach einem der vorangehenden Ansprüche,
wobei die Erfassungseinheit eine Signaleinheit umfasst, welche Signaleinheit das Verbinden der Programmiereinheit mit der Erfassungseinheit und/oder das Übermitteln des ersten Kodierungssignal indiziert.

4. Verfahren nach einem der vorangehenden Ansprüche,
wobei die Erfassungssteuereinheit dazu ausgebildet ist, das erste Kodierungssignal von physiologischen Signalen zu unterscheiden.

5. Verfahren nach einem der vorangehenden Ansprüche,
wobei das Übermitteln des ersten Kodierungssignals ein Ausgeben eines zeitlich veränderten elektrischen Stromes umfasst.

6. Verfahren zu einer Einstellung einer Übertragungsfrequenz einer physiologischen Überwachungseinheit umfassend eine Erfassungseinheit und eine Empfängereinheit,
wobei die Erfassungseinheit und die Empfängereinheit drahtlos miteinander verbunden sind und dazu ausgebildet sind, auf der Übertragungsfrequenz Daten auszutauschen,
die Erfassungseinheit eine Sensoreinheit und eine Erfassungssteuereinheit umfasst, und
die Sensoreinheit zur Aufnahme physiologischer Signale eines Untersuchungsobjekts ausgebildet ist,
mittels einer Programmiereinheit umfassend eine Kopplungseinheit gemäß den folgenden Verfahrensschritten:
- Bereitstellen der Übertragungsfrequenz durch die Programmiereinheit,
- Verbinden der Programmiereinheit mit der Erfassungseinheit umfassend ein Anschließen der Kopplungseinheit an die Sensoreinheit,
- Übermitteln eines ersten Kodierungssignal repräsentativ für die Übertragungsfrequenz von der Programmiereinheit an die Erfassungseinheit,
- Bereitstellen der Übertragungsfrequenz an die Empfängereinheit mittels Eingabe durch einen Benutzer oder gemäß den folgenden Verfahrensschritten:
- Verbinden der Programmiereinheit mit einem von der Empfängereinheit umfassten Eingang, und
- Übermitteln eines zweiten Kodierungssignals repräsentativ für die Übertragungsfrequenz von der Programmiereinheit an eine von der Empfängereinheit umfasste Empfängersteuereinheit
wobei die Sensoreinheit dazu ausgebildet ist, einen Puls eines Untersuchungsobjektes zu erfassen, und die Sensoreinheit einen Fingerclip umfasst, die Kopplungseinheit eine Form eines Fingers aufweist und das Verbinden der Programmiereinheit mit der Erfassungseinheit ein Umschließen der Kopplungseinheit durch den Fingerclip umfasst.

7. Verfahren nach Anspruch 6,
wobei das Übermitteln des ersten Kodierungssignals das Ausgeben eines zeitlich veränderten Lichtsignals umfasst.

8. Verfahren nach einem der Ansprüche 6 bis 7,
wobei das erste Kodierungssignal ein Triggersignal umfasst, und das Übermitteln des ersten Kodierungssignals eine Signalisierung einer beabsichtigten Änderung der Übertragungsfrequenz an die Erfassungseinheit durch das Triggersignal umfasst.

9. Verfahren nach einem der Ansprüche 6 bis 8,
wobei die Erfassungseinheit eine Signaleinheit umfasst, welche Signaleinheit das Verbinden der Programmiereinheit mit der Erfassungseinheit und/oder das Übermitteln des ersten Kodierungssignal indiziert.

10. Verfahren nach einem der Ansprüche 6 bis 9,
wobei die Erfassungssteuereinheit dazu ausgebildet ist, das erste Kodierungssignal von physiologischen Signalen zu unterscheiden.

11. Verfahren zu einer Einstellung einer Übertragungsfrequenz einer physiologischen Überwachungseinheit umfassend eine Erfassungseinheit und eine Empfängereinheit,
wobei die Erfassungseinheit und die Empfängereinheit drahtlos miteinander verbunden sind und dazu ausgebildet sind, auf der Übertragungsfrequenz Daten auszutauschen,
die Erfassungseinheit eine Sensoreinheit und eine Erfassungssteuereinheit umfasst, und
die Sensoreinheit zur Aufnahme physiologischer Signale eines Untersuchungsobjekts ausgebildet ist,
mittels einer Programmiereinheit umfassend eine Kopplungseinheit gemäß den folgenden Verfahrensschritten:
- Bereitstellen der Übertragungsfrequenz durch die Programmiereinheit,
- Verbinden der Programmiereinheit mit der Erfassungseinheit umfassend ein passgenaues Anschließen der Kopplungseinheit an die Sensoreinheit,
- Übermitteln eines ersten Kodierungssignal repräsentativ für die Übertragungsfrequenz von der Programmiereinheit an die Erfassungseinheit,
- Bereitstellen der Übertragungsfrequenz an die Empfängereinheit gemäß den folgenden Verfahrensschritten:
- Verbinden der Programmiereinheit mit einem von der Empfängereinheit umfassten Eingang, und
- Übermitteln eines zweiten Kodierungssignals repräsentativ für die Übertragungsfrequenz von der Programmiereinheit an eine von der Empfängereinheit umfasste Empfängersteuereinheit,
wobei das erste Kodierungssignal ein Triggersignal umfasst, das Übermitteln des ersten Kodierungssignals eine Signalisierung einer beabsichtigten Änderung der Übertragungsfrequenz an die Erfassungseinheit durch das Triggersignal umfasst, und
die Erfassungseinheit dazu ausgebildet ist, bei Erkennen des Triggersignals einen Status anzunehmen, in welchem eine Änderung der Übertragungsfrequenz zulässig ist und eine Aufnahme physiologischer Signale unterbrochen wird.

12. Verfahren nach Anspruch 11,
wobei die Erfassungseinheit eine Signaleinheit umfasst, welche Signaleinheit das Verbinden der Programmiereinheit mit der Erfassungseinheit und/oder das Übermitteln des ersten Kodierungssignal indiziert.

13. Verfahren nach einem der Ansprüche 11 bis 12,
wobei die Erfassungssteuereinheit dazu ausgebildet ist, das erste Kodierungssignal von physiologischen Signalen zu unterscheiden.

14. Verfahren zum Erfassen eines physiologischen Signals mittels einer physiologischen Überwachungseinheit umfassend eine Erfassungseinheit und eine Empfängereinheit, wobei die Erfassungseinheit und die Empfängereinheit drahtlos miteinander verbunden sind und dazu ausgebildet sind, auf der Übertragungsfrequenz Daten auszutauschen,
gemäß den folgenden Verfahrensschritten:
- Detektion des physiologischen Signals mittels der Erfassungseinheit,
- Drahtloses Übertragen des physiologischen Signals von der Erfassungseinheit auf die Empfängereinheit auf der Übertragungsfrequenz,
- Verarbeitung des physiologischen Signals durch die Empfängereinheit,
wobei die Übertragungsfrequenz der physiologischen Überwachungseinheit gemäß einer Ausführung eines Verfahrens nach einem der vorangehenden Ansprüche eingestellt wird.

15. Physiologisches Überwachungssystem umfassend eine Programmiereinheit und eine physiologische Überwachungseinheit umfassend eine Erfassungseinheit und eine Empfängereinheit, welche zu einer Ausführung eines Verfahrens nach einem der Ansprüche 1 bis 13 ausgelegt ist.

## Claims

1. Method for adjusting a transmission frequency of a physiological monitoring unit comprising a capture unit and a receiver unit,
wherein the capture unit and the receiver unit are wirelessly connected together and are designed to exchange data on the transmission frequency,
the capture unit comprises a sensor unit and a capture control unit, and
the sensor unit is designed to record physiological signals of an examination object,
by means of a programming unit comprising a coupling unit in accordance with the following method steps:
- providing the transmission frequency by means of the programming unit,
- connecting the programming unit to the capture unit comprising an attachment of the coupling unit to the sensor unit,
- transferring a first coding signal representative of the transmission frequency from the programming unit to the capture unit,
- providing the transmission frequency to the receiver unit by means of input by a user or in accordance with the following method steps:
- connecting the programming unit to an input comprised by the receiver unit, and
- transferring a second coding signal representative of the transmission frequency from the programming unit to a receiver control unit comprised by the receiver unit,
wherein the sensor unit comprises at least two electrodes and is designed to capture an electrocardiogram, the coupling unit comprises at least one pin,
and the attachment of the coupling unit to the sensor unit includes a connection of the at least one pin to one of the at least two electrodes.

2. Method according to claim 1,
wherein the first coding signal comprises a trigger signal, and the transfer of the first coding signal comprises a signalling to the capture unit by the trigger signal of an intended change of the transmission frequency.

3. Method according to one of the preceding claims,
wherein the capture unit comprises a signal unit, which signal unit indicates the connection of the programming unit to the capture unit and/or the transfer of the first coding signal.

4. Method according to one of the preceding claims,
wherein the capture control unit is designed to distinguish the first coding signal from physiological signals.

5. Method according to one of the preceding claims,
wherein the transfer of the first coding signal comprises an output of a temporally changing electric current.

6. Method for adjusting a transmission frequency of a physiological monitoring unit comprising a capture unit and a receiver unit,
wherein the capture unit and the receiver unit are wirelessly connected together and are designed to exchange data on the transmission frequency,
the capture unit comprises a sensor unit and a capture control unit, and
the sensor unit is designed to record physiological signals of an examination object,
by means of a programming unit comprising a coupling unit in accordance with the following method steps:
- providing the transmission frequency by means of the programming unit,
- connecting the programming unit to the capture unit comprising an attachment of the coupling unit to the sensor unit,
- transferring a first coding signal representative of the transmission frequency from the programming unit to the capture unit,
- providing the transmission frequency to the receiver unit by means of input by a user or in accordance with the following method steps:
- connecting the programming unit to an input comprised by the receiver unit, and
- transferring a second coding signal representative of the transmission frequency from the programming unit to a receiver control unit comprised by the receiver unit,
wherein the sensor unit is designed to capture a pulse of an examination object and the sensor unit comprises a finger clip, the coupling unit has a shape of a finger, and the connection of the programming unit to the capture unit comprises the coupling unit being surrounded by the finger clip.

7. Method according to claim 6,
wherein the transfer of the first coding signal comprises the output of a temporally changing light signal.

8. Method according to one of claims 6 to 7,
wherein the first coding signal comprises a trigger signal, and the transfer of the first coding signal comprises a signalling to the capture unit by the trigger signal of an intended change of the transmission frequency.

9. Method according to one of claims 6 to 8,
wherein the capture unit comprises a signal unit, which signal unit indicates the connection of the programming unit to the capture unit and/or the transfer of the first coding signal.

10. Method according to one of claims 6 to 9,
wherein the capture control unit is designed to distinguish the first coding signal from physiological signals.

11. Method for adjusting a transmission frequency of a physiological monitoring unit comprising a capture unit and a receiver unit,
wherein the capture unit and the receiver unit are wirelessly connected together and are designed to exchange data on the transmission frequency,
the capture unit comprises a sensor unit and a capture control unit, and
the sensor unit is designed to record physiological signals of an examination object,
by means of a programming unit comprising a coupling unit in accordance with the following method steps:
- providing the transmission frequency by means of the programming unit,
- connecting the programming unit to the capture unit comprising an attachment of the coupling unit to the sensor unit with a precise fit,
- transferring a first coding signal representative of the transmission frequency from the programming unit to the capture unit,
- providing the transmission frequency to the receiver unit in accordance with the following method steps:
- connecting the programming unit to an input comprised by the receiver unit, and
- transferring a second coding signal representative of the transmission frequency from the programming unit to a receiver control unit comprised by the receiver unit,
wherein the first coding signal comprises a trigger signal, the transfer of the first coding signal comprises a signalling to the capture unit by the trigger signal of an intended change of the transmission frequency, and
the capture unit is designed, upon acknowledging the trigger signal, to assume a status in which a change of the transmission frequency is permissible and a recording of physiological signals is interrupted.

12. Method according to claim 11,
wherein the capture unit comprises a signal unit, which signal unit indicates the connection of the programming unit to the capture unit and/or the transfer of the first coding signal.

13. Method according to one of claims 11 to 12,
wherein the capture control unit is designed to distinguish the first coding signal from physiological signals.

14. Method for capturing a physiological signal by means of a physiological monitoring unit comprising a capture unit and a receiver unit, wherein the capture unit and the receiver unit are wirelessly connected together and are designed to exchange data on the transmission frequency data,
in accordance with the following method steps:
- detecting the physiological signal by means of the capture unit,
- wirelessly transmitting the physiological signal from the capture unit to the receiver unit on the transmission frequency,
- processing of the physiological signal by the receiver unit,
wherein the transmission frequency of the physiological monitoring unit was previously adjusted in accordance with an embodiment of a method according to one of the preceding claims.

15. Physiological monitoring system comprising a programming unit and a physiological monitoring unit comprising a capture unit and a receiver unit, which is configured to execute a method according to one of claims 1 to 13.

## Revendications

1. Procédé de réglage d'une fréquence de transmission d'une unité de contrôle physiologique comprenant une unité de détection et une unité de réception,
dans lequel l'unité de détection et l'unité de réception sont reliées l'une à l'autre sans fil et sont constituées pour échanger des données à la fréquence de transmission,
l'unité de détection comprend une unité de capteur et une unité de commande de détection, et
l'unité de capteur est constituée pour l'enregistrement de signaux physiologiques d'un objet à examiner,
au moyen d'une unité de programmation comprenant une unité de liaison selon les stades de procédé suivants :
- mise à disposition de la fréquence de transmission par l'unité de programmation,
- liaison de l'unité de programmation à l'unité de détection comprenant une connexion de l'unité de liaison à l'unité de capteur,
- transmission d'un premier signal de codage, représentatif de la fréquence de transmission, de l'unité de programmation à l'unité de détection,
- mise de la fréquence de transmission à disposition de l'unité de réception au moyen d'une entrée par un utilisateur ou selon les stades de procédé suivants :
- liaison de l'unité de programmation à une entrée comprise par l'unité de réception et,
- transmission d'un deuxième signal de codage, représentatif de la fréquence de transmission, de l'unité de programmation à une unité de commande de réception comprise par l'unité de réception,
dans lequel l'unité de capteur comprend au moins deux électrodes et est constituée pour relever un électrocardiogramme, l'unité de liaison comprend au moins une broche,
et la connexion de l'unité de liaison à l'unité de capteur comprend une liaison de la au moins une broche à l'une des au moins deux électrodes.

2. Procédé suivant la revendication 1,
dans lequel le premier signal de codage comprend un signal de déclenchement et la transmission des premiers signaux de codage comprend une signalisation d'une modification envisagée de la fréquence de transmission à l'unité de détection par le signal de déclenchement.

3. Procédé suivant l'une des revendications précédentes,
dans lequel l'unité de détection comprend une unité de signal, laquelle unité de signal indique la liaison de l'unité de programmation à l'unité de détection et/ou la transmission du premier signal de codage.

4. Procédé suivant l'une des revendications précédentes,
dans lequel l'unité de commande de détection est constituée pour distinguer le premier signal de codage de signaux physiologiques.

5. Procédé suivant l'une des revendications précédentes,
dans lequel la transmission du premier signal de codage comprend l'émission d'un courant électrique variable dans le temps.

6. Procédé de réglage d'une fréquence de transmission d'une unité de contrôle physiologique comprenant une unité de détection et une unité de réception,
dans lequel l'unité de détection et l'unité de réception sont reliées l'une à l'autre sans fil et sont constituées pour échanger des données à la fréquence de transmission,
l'unité de détection comprend une unité de capteur et une unité de commande de détection, et
l'unité de capteur est constituée pour l'enregistrement de signaux physiologiques d'un objet à examiner,
au moyen d'une unité de programmation comprenant une unité de liaison selon les stades de procédé suivants :
- mise à disposition de la fréquence de transmission par l'unité de programmation,
- liaison de l'unité de programmation à l'unité de détection comprenant une connexion de l'unité de liaison à l'unité de capteur,
- transmission d'un premier signal de codage, représentatif de la fréquence de transmission, de l'unité de programmation à l'unité de détection,
- mise de la fréquence de transmission à disposition de l'unité de réception au moyen d'une entrée par un utilisateur ou selon les stades de procédé suivants :
- liaison de l'unité de programmation à une entrée comprise par l'unité de réception et
- transmission d'un deuxième signal de codage, représentatif de la fréquence de transmission, de l'unité de programmation à une unité de commande de réception comprise par l'unité de réception,
dans lequel l'unité de capteur est constituée pour détecter une impulsion d'un objet à examiner et l'unité de capteur comprend un clip de doigt, l'unité de liaison a la forme d'un doigt et la liaison de l'unité de programmation à l'unité de détection comprend un entourage de l'unité de liaison par le clip de doigt.

7. Procédé suivant la revendication 6,
dans lequel la transmission du premier signal de codage comprend l'émission d'un signal lumineux variable dans le temps.

8. Procédé suivant l'une des revendications 6 à 7,
dans lequel le premier signal de codage comprend un signal de déclenchement et la transmission des premiers signaux de codage comprend une signalisation d'une modification envisagée de la fréquence de transmission à l'unité de détection par le signal de déclenchement.

9. Procédé suivant l'une des revendications 6 à 8,
dans lequel l'unité de détection comprend une unité de signal, laquelle unité de signal indique la liaison de l'unité de programmation à l'unité de détection et/ou la transmission du premier signal de codage.

10. Procédé suivant l'une des revendications 6 à 9,
dans lequel l'unité de commande de détection est constituée pour distinguer le premier signal de codage de signaux physiologiques.

11. Procédé de réglage d'une fréquence de transmission d'une unité de contrôle physiologique comprenant une unité de détection et une unité de réception,
dans lequel l'unité de détection et l'unité de réception sont reliées l'une à l'autre sans fil et sont constituées pour échanger des données à la fréquence de transmission,
l'unité de détection comprend une unité de capteur et une unité de commande de détection, et
l'unité de capteur est constituée pour l'enregistrement de signaux physiologiques d'un objet à examiner,
au moyen d'une unité de programmation comprenant une unité de liaison selon les stades de procédé suivants :
- mise à disposition de la fréquence de transmission par l'unité de programmation,
- liaison de l'unité de programmation à l'unité de détection comprenant une connexion de l'unité de liaison à l'unité de capteur,
- transmission d'un premier signal de codage, représentatif de la fréquence de transmission, de l'unité de programmation à l'unité de détection,
- mise de la fréquence de transmission à disposition de l'unité de réception selon les stades de procédé suivants :
- liaison de l'unité de programmation à une entrée comprise par l'unité de réception et
- transmission d'un deuxième signal de codage, représentatif de la fréquence de transmission, de l'unité de programmation à une unité de commande de réception comprise par l'unité de réception,
dans lequel le premier signal de codage comprend un signal de déclenchement, la transmission du premier signal de codage comprend une signalisation d'une modification envisagée de la fréquence de transmission à l'unité de détection par le signal de déclenchement, et
l'unité de détection est constituée pour prendre à la détection du signal de déclenchement un statut, dans lequel une modification de la fréquence de transmission est admissible et un enregistrement de signaux physiologiques est interrompu.

12. Procédé suivant la revendication 11,
dans lequel l'unité de détection comprend une unité de signal, laquelle unité de signal indique la liaison de l'unité de programmation à l'unité de détection et/ou la transmission du premier signal de codage.

13. Procédé suivant l'une des revendications 11 à 12,
dans lequel l'unité de commande de détection est constituée pour distinguer le premier signal de codage de signaux physiologiques.

14. Procédé de réglage d'une fréquence de transmission d'une unité de contrôle physiologique comprenant une unité de détection et une unité de réception, dans lequel l'unité de détection et l'unité de réception sont reliées l'une à l'autre sans fil et sont constituées pour échanger des données à la fréquence de transmission,
selon les stades de procédé suivants :
- détection du signal physiologique au moyen de l'unité de détection,
- transmission sans fil du signal physiologique de l'unité de détection à l'unité de réception à la fréquence de transmission,
- traitement du signal physiologique par l'unité de réception,
dans lequel on règle la fréquence de transmission de l'unité de contrôle physiologique en effectuant un procédé suivant l'une des revendications précédentes.

15. Système de contrôle physiologique, comprenant une unité de programmation et une unité de contrôle physiologique, comprenant une unité de détection et une unité de réception, qui est conçue pour effectuer un procédé suivant l'une des revendications 1 à 13.
